# NEUE EUROPÄISCHE PATENTSCHRIFT

(11) **EP 0 902 269 B2**
(45) Veröffentlichungstag und Bekanntmachung des Hinweises auf die Entscheidung über den Einspruch: **09.07.2008**
(45) Hinweis auf die Patenterteilung: 02.10.2002
(21) Anmeldenummer: 98114022.1
(22) Anmeldetag: 27.07.1998
(51) Int. Cl.: G01N 11/14, G01N 33/26, G01N 33/32, G01N 33/34

(54) **Verfahren zur Bestimmung der Immobilisierung kolloider Beschichtungsdispersionen**
Method for the determination of the immobilisation of colloid coating dispersions
Méthode pour la détermination de l'immobilisation de dispersions colloidales de revêtement

(30) Priorität: 30.07.1997 DE 19732877
(43) Veröffentlichungstag der Anmeldung: 17.03.1999
(73) Patentinhaber: BASF SE, 67056 Ludwigshafen (DE)
(72) Erfinder: Willenbacher, Norbert Dr., 67292 Kirchheimbolanden (DE); Hanciogullari, Harutyun Dr., 67117 Limburgerhof (DE); Rädle, Matthias Dr., 67273 Weisenheim (DE); Ettmüller, Jürgen, 67454 Hassloch (DE)
(74) Vertreter: Isenbruck, Günter

(56) Entgegenhaltungen:
- WO-A-94/08222
- WO-A-96/16323
- DE-A- 4 133 439
- NL-A- 7 713 735
- US-A- 5 056 358
- US-A- 5 631 409
- Bohlin Reologi drawing Nr.332042 dated 881004
- Malvern Instruments website extracts "Corporate history"
- Römpp Chemie Lexikon 9.Auflage, Seite 400
- JL Duda et al., Society of Petroleum Engineers Journal 21 (1981) 613-622
- B. Kalpakci et al., ASLE Transactions 25 (1981) 283-288

## Beschreibung

Die Erfindung betrifft ein Verfahren zur Bestimmung der Immobilisierung kolloider Beschichtungsdispersionen, die auf ein poröses Su strat aufgetragen werden sowie die Verwendung des Verfahrens.

Die Kenntnis der zeitlichen Veränderung von Eigenschaften kolloider Beschichtungsdispersionen während des Auftrags auf poröse Substrate ist eine wesentliche Voraussetzung für die Verbesserung der technischen Verarbeitbarkelt sowie die Optimierung neuer Rezepturen. Von besonderem Interesse ist in diesem Zusammenhang die Veränderung der Viskosität der kolloiden Beschichtungsdispersion während des Auftragvorganges und Insbesondere die Kenntnis des Zeitpunkts, zu dem die Viskosität plötzlich ansteigt. Der Knickpunkt in der graphischen Darstellung der zeitabhängigen Viskositätsänderung wird als Immobillislerungspunkt, die entsprechende Zeit als Immobilisierungszeit bezeichnet. Als Immobilisierung wird der Zustand bezeichnet, bei dem aufgrund des erhöhten Feststoffgehaltes alle das Feststoffsystem betreffenden Fließ- und Transportprozesse unterbunden sind.

Anwendungsgebiet ist insbesondere die Papierfabrikation, wo die Kenntnis der Immobilisierung von Papierstreichfarben während des Auftragsvorgangs auf Rohpapier für die technische Nutzbarkeit neuer Rezepturen eine bedeutende Rolle spielt.

Die Immobilisierung kolloider Beschichtungsdispersionen ist jedoch auch auf anderen Gebieten von Bedeutung, beispielsweise für Klebstoffe oder Mörtel, wobei die flüssige Phase der Beschichtungsdispersion nicht auf Wasser beschränkt sein muß.

Ein gebräuchliches Verfahren zur Charakterisierung der Immobilisierung ist die sogenannte Tonteller-Methode (M. Baumeister und J. Weigl, Wochenblatt für Papierfabrikation 108(5) (1980), 145-151). Danach wird ein Tropfen einer Papierstreichfarbe auf einen unglasierten Tonteller gegeben und mit einem Spatel so lange gerührt, bis die Masse fest ist. Die erforderliche Zeit wird als Immobillsierungszeit bezeichnet.

Das Verfahren hat die Nachteile, daß die Immobilisierung nicht unter verarbettungsrelevanten Bedingungen bestimmt wird - das flüssigkeitsaufnehmende Substrat ist vorgegeben, es kann keine definierte Schichtdicke vorgegeben werden - und die Immobllisierungsbestimmung ist subjektiv.

Das obengenannte Verfahren wurde durch das Reflexionsrnessungsverfahren [U.Beck et al., Wochenblatt für Papierfabrikation 111 (16) (1983), 561-565)] automatisiert und objektiviert. Die Versuchsanordnung wird durch eine Photodiode ergänzt, die die zeitliche Entwicklung der Intensität der Reflexion eines Strahlenbündels an der Oberfläche der Papierstreichfarbe mißt. Auch diese Versuchsanordnung simuliert jedoch nicht praxisrelevante Bedingungen.

Nach der Gradek-Methode (S.E. Sandas. P.J. Salminen, D.E Eklund, Tappi 72(12) (1989), 207-210) wird die In einer bestimmten Zeit und unter bestimmtem Druck ein definiertes Rohpapier penetrierende Wassermenge gravimetrisch bestimmt. Es wird somit nicht die Immobilisierung, sondern der Wasserverlust durch Penetration in das Rohpapier, mit dem die Immobilisierung verbunden ist, gemessen. Das Verfahren simuliert nur ansatzweise praxisrelevante Bedingungen: Farbe und Rohpapier sind durch ein Polycarbonat-Filter getrennt, es findet keine Scherbeanspruchung statt, und es wird keine prozeßrelevante Schichtdicke eingestellt.

Ein spezielles Filtrationsverfahren sowie eine angepaßte Vorrichtung wurden darüber hinaus entwickelt (J. Ramthun, L. Reif, J. Röttger-Heinz, J. Waldi und G. Wallpott, Wochenblatt für Papierfabrikation 122(19) (1994), 745-750): Bei vorgegebenem Druck und unter Rühren wird die Papierstreichfarbe durch einen Filter mit definierter Porengröße filtriert, wobei auch Rohpapier als Filter eingesetzt werden kann. Der drehzahlregelbare Rührer kann über einen Mikrometer in der Höhe über dem Filter variiert werden. Der Wasserdurchgang durch den Filter wird gravimetrisch bestimmt. Zusätzlich wird die zeitliche Entwicklung der Leistungsaufnahme des Rührers gemessen und dient als Maß für den mit dem Wasserverlust verbundenen Anstieg der Viskosität. Das Verfahren hat den Nachteil, daß keine laminare Scherströmung unter dem Rührer sowie keine prozeßrelevante Schichtdicke eingestellt werden können, da auch die Schicht oberhalb der unteren Rührerkante entwässert wird.

Aufgabe der Erfindung ist es daher, ein Verfahren bereitzustellen, wonach die Immobilisierung kolloider Beschichtungsdispersionen beim Aufbringen auf ein poröses Substrat unter Bedingungen bestimmt werden kann, die für den Verarbeitungsprozeß relevant sind: Die Schichtdicke der Beschichtungsdispersion sowie die beim Beschichtungsvorgang auftretenden Scherbeanspruchungen können den praxisrelevanten Bedingungen angenähert werden; die Vorrichtung zur Durchführung des Verfahrens ist robust und einfach zu handhaben, und die erforderlichen technischen Einrichtungen können als Zusatzmodule in handelsübliche Rotationsrheometer integriert werden.

Aus US 5,631,409 ist ein Rotationsrheometer bekannt, dessen stationäre oder rotierende Platte zwecks verbesserter Wechselwirkung mit dem rheologisch zu untersuchenden Medium porös ausgebildet sein kann. Die Druckschrift enthält keinen Hinweis darauf, die poröse Platte als Substrat auszubilden, das durch Aufnahme eines Teils der flüssigen Phase einer Beschichtungsdispersion die Immobilisierung desselben verurscht. Die Aufgabe wird erfindungsgemäß gelöst durch ein Verfahren zur Bestimmung der Immobilisierung kolloider Beschichtungsdispersionen beim Aufbringen auf ein poröses Substrat durch Viskositätsmessung, wonach auf das poröse Substrat, das eine Begrenzungsfläche des Meßspalts eines Rheometers bildet, die kolloide Beschichtungsdispersion aufgebracht und die zeitliche Änderung der Viskosität gemessen wird.

In bevorzugter Weise kann die Viskositätsmessung unter Vorgabe einer konstanten Schubspannung oder Schergeschwindigkeit erfolgen.

Das erfindungsgemäße Verfahren ist besonders geeignet zur Charakterisierung der Immobilisierung von Papierstreichfarbe durch Penetration von Wasser in das zu beschichtende Rohpapier. Das Verfahren eignet sich jedoch ebenso zur Charakterisierung anderer kolloider Beschichtungsdispersionen, beispielsweise von Klebstoffen oder Mörtel. In diesen Fällen wird das jeweils entsprechende poröse Substrat (z. B. Tonplatte, Glasfritte) anstelle des Rohpapiers als Rheometergrundplatte eingesetzt.

Die Schichtdicke des porösen Substrats und somit die Höhe des Rheometer-Meßspalts kann bevorzugt auf einen Wert zwischen 20µm und 2mm, besonders bevorzugt zwischen 50µm und 500µm eingestellt werden.

Gemäß einer bevorzugten Ausführungsform kann durch Anlegen eines Unterdrucks an die Fläche des porösen Substrats, die der mit der kolloiden Beschichtungsdispersion beaufschlagten Fläche gegenüberliegt, zusätzlich die Druckbeanspruchung den praxisrelevanten Bedingungen angenähert werden.

In vorteilhafter Weise kann simultan zur Charakterisierung der zeitlichen Entwicklung der Viskosität der zeitliche Verlauf des Verlustes an flüssiger Phase in der kolloiden Beschichtungsdispersion, bevorzugt mittels eines faseroptischen Streulichtmeßsystems, verfolgt werden. Der Verlust an flüssiger Phase kann jedoch auch durch andere bekannte Meßmethoden, insbesondere durch dielektrische und lichtspektroskopische Methoden verfolgt werden.

Erfindungsgemäß wird der zeitliche Verlauf der Viskositätsänderung mit einem handelsüblichen schubspannungskontrollierten Rheometer gemessen, das in der Weise modifiziert wird, daß das poröse Substrat, das durch die Aufnahme eines Teils der flüssigen Phase die Immobilisierung verursacht, eine Begrenzungsfläche des Rheometerspalts bildet.

Besonders geeignet sind Rheometer mit Platte-Platte-Geometrie, es sind jedoch auch Rheometer-Vorrichtungen möglich, die eine Platte-Kegel- oder eine Zylinder-Anordnung aufweisen.

Die Erfindung wird im folgenden durch die Zeichnung und die Ausführungsbeispiele näher erläutert.

Es zeigen im einzelnen:
- Figur 1: die schematische Darstellung eines für die Immobilisierungsmessungen modifizierten handelsüblichen Rotationsrheometers,
- Figur 2: die schematische Darstellung eines Rotationsrheometers nach Figur 1 mit zusätzlicher integrierter NIR-Sonde zur Wassergehaltsbestimmung, in Seitenansicht (Figur 2a) und Aufsicht (Figur 2b),
- Figur 3: Meßergebnisse der scheinbaren Viskosität als Funktion der Entwässerungszeit für verschiedene Verdickerkonzentrationen,
- Figur 4: die Kalibrierungskurve des Ausgangssignals des NIR-Detektors-für Streichfarbe B und
- Figur 5: Meßergebnisse der simultanen Bestimmung von scheinbarer Viskosität und Feststoffgehalt als Funktion der Entwässerungszeit für Streichfarbe B.

### BEISPIELE

### BEISPIEL 1

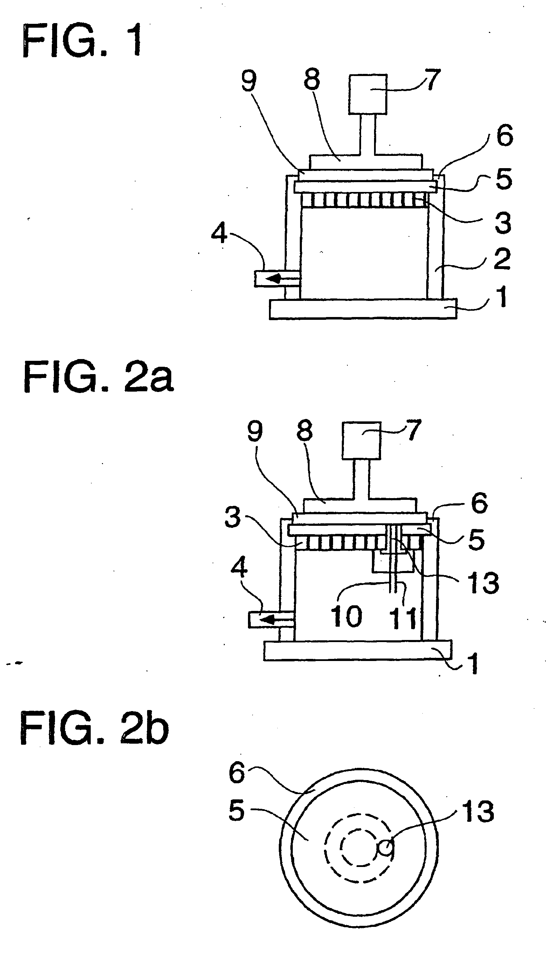

Ein handelsübliches, schubspannungskontrolliertes Rotationsrheometer, beispielsweise Bohlin^{R} CS mit einem Probenaufnahme- oder Meßspalt in Platte-Platte-Geometrie wird folgendermaßen modifiziert (Figur 1):
Auf die feststehende Rheometer-Grundplatte 1 wird ein zylinderförmiger, metallischer Hohlkörper 2 aufgesetzt, dessen Deckfläche aus einer gelochten Metallplatte 3 besteht. In dessen Mantelfläche ist ein Stutzen 4 eingelassen, der zum Anschluß einer Vakuumpumpe dient. Auf der gelochten Metallplatte 3 wird das poröse Substrat 5, beispielsweise Rohpapier, mit Hilfe eines geeigneten Klemmrings 6 befestigt. Dieses poröse Substrat 5 dient nun als untere Begrenzungsfläche des Meßspalts. Die gegenüberliegende Begrenzungsfläche ist eine mit dem Antriebs- und Detektionssystems 7 des Rheometers verbundene Metallplatte 8. Der dazwischenliegende Spalt wird mit der zu untersuchenden kolloiden Beschichtungsdispersion 9, beispielsweise mit Papierstreichfarbe, befüllt.

Mit dieser Anordnung können Viskositätsmessungen analog zum Vorgehen bei einem Meßspalt mit konventioneller Grundplatte durchgeführt werden. Die Spalthöhe kann zwischen 20 µm und 2 mm, bevorzugt zwischen 50 und 500 µm variiert werden.

Das Wasser aus der Streichfarbe 9 wird vom Rohpapier 5 aufgesaugt, bevorzugt durch Anlegen eines geeigneten Unterdrucks, insbesondere im Bereich von 200 bis 500 mbar. Dadurch wird die Streichfarbe 9, ähnlich wie beim Beschichtungsprozeß bei der Papierveredlung, aufkonzentriert. Der mit diesem Vorgang verknüpfte Anstieg der Viskosität wird bei Vorgabe einer konstanten Schubspannung, bevorzugt 0,1 bis 1000 Pa, besonders bevorzugt 10 bis 100 Pa, gemessen.

Der zeitliche Verlauf dieses Viskositätsanstiegs charakterisiert die Immobilisierungskinetik, die vom Wasserrückhaltevermögen der jeweiligen Papierstreichfarbe und damit von deren Rezeptur sowie von der Strukturbildung während des Beschichtungs- und Entwässerungsvorgangs abhängt.

Durch Variation der vorgegebenen Schubspannung und gegebenenfalls des Unterdrucks können unterschiedliche Beschichtungsbedingungen simuliert werden.

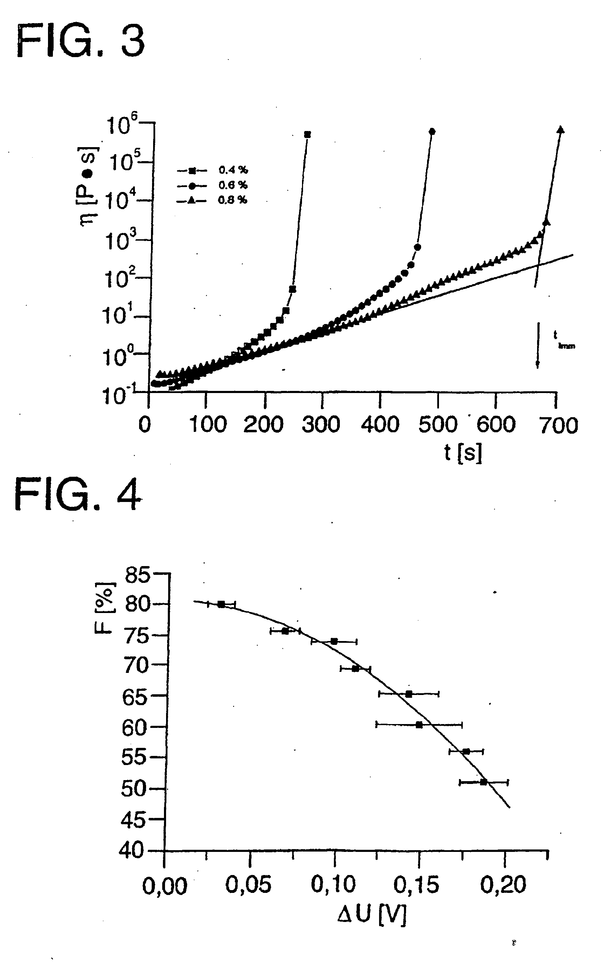

Damit aufgrund des Wasserverlustes während der Messung der Kontakt zwischen Streichfarbe 9 und Metallplatte 8 nicht abreißt, wird der Spalt überfüllt (siehe Figur 1), so daß von außen Farbe in den Spalt nachströmen kann. Daher ist der äußere Bereich des Meßspalts immer feuchter als dessen Inneres, d.h. die Probe ist inhomogen und die gemessenen Viskositäten müssen als scheinbare oder relative Werte angesehen werden. Der Knickpunkt in der halblogarithmischen Darstellung der Viskosität als Funktion der Zeit (Figur 3) wird als Immobilisierungspunkt, die entsprechende Zeit als Immoblisierungszeit tᵢₘₘ bezeichnet. Typischerweise findet man nach der Immobilisierung im inneren Bereich des Probenspalts einen festkörperartigen Filterkuchen mit einem Rest-Wassergehalt von 15-20% vor.

Das oben beschriebene Meßverfahren ist nicht auf die Charakterisierung der Immobilisierung von Papierstreichfarbe durch Penetration von Wasser in das zu beschichtende Rohpapier beschränkt. Analog können auch andere (auch nichtwässrige) Beschichtungsdispersionen (z.B. Klebstoffe, Mörtel) untersucht werden. An Stelle des Rohpapiers kann das jeweilige Substrat (z.B. Tonplatte, Glasfritte) als Rheometergrundplatte eingesetzt werden.

### BEISPIEL 2

Die Vorrichtung nach Beispiel 1 wird verbessert durch Integrieren eines faseroptischen Streulichtmeßsystems nach EP-B1-0 472 899 in die gelochte Metallplatte 3 des modifizierten Rheometers (Figur 2). Dadurch wird simultan zur Viskositätsbestimmung die Messung des zeitlichen Verlaufs des Wassergehalts möglich.

Das durch eine breitbandige Lichtquelle 10, vorzugsweise Halogenlichtquelle, in eine OH-arme Quarzfaser, vorzugsweise einem Faserbündel aufgebaut aus 200 µm-Fasern, zum Meßort geleiteten Licht wird am Produkt gestreut, in die parallel liegenden Empfangsfasern rückgestreut und zum Detektor 11 geleitet. Die Sensorspitze 13 des faseroptischen Streulichtmeßsystems ragt durch die gelochte Metallplatte 3 und das poröse Substrat 5 in die kolloide Beschichtungsdispersion 9.

Die Anordnung der Remission mit einem definierten Winkel zwischen Sender/ Empfängerund der Meßebene von vorzugsweise 11° erlaubt bei den untersuchten Konzentrationen eine reflexarme Messung der diffusen Rückstreuung durch das Produkt bei einer Eindringtiefe von wenigen µm.

Die Absorption der NIR-Strahlung durch das Wasser wird durch die Anregung der 2. Harmonischen der OH-Schwingung verursacht. Das Licht wird zur Erreichung der erforderlichen Signalhöhe breitbandig bei 1470±20 nm aufgenommen. Zur Kalibrierung der Produktstreuung ohne Wasserabsorption wird gleichzeitig eine Messung in einem nahe der Absorptionsbande liegenden aber nicht absorbierenden Wellenlängenbereich des Spektrums, vorzugsweise bei 1300 nm oder 1700 nm, durchgeführt. Um gleichzeitig Messungen bei mehreren Wellenlängen durchführen zu können, wird das Licht nicht auf der Sendeseite sondern auf der Nachweisseite selektiert.

Zusätzlich wird die Intensität der Lichtquelle bei den ausgewählten Wellenlängen (hier 1470 nm und 1700 nm) bestimmt. Dadurch können Signalschwankungen aufgrund der Drift der Lichtquelle eliminiert werden. Die Stabilität der übrigen Komponenten wird bauseitig garantiert.

Durch geeignete Bohrungsführung um die mit 4 mm Durchmesser möglichst klein gehaltene Meßfläche wird die Entwässerung direkt am Meßort nur wenig behindert. Aufgrund der aufgeprägten Scherung wird das Produkt an der wandgängig in der Ebene des porösen Substrats 5 liegenden Sensorspitze 13 vorbeigeführt, wobei eine Mittelung über das gesamte auf dem entsprechenden Kreisring befindliche Produkt erreicht wird. An der Meßstelle ist eine passende Lochung des Papiers notwendig, um das Licht direkt in die Beschichtungsdispersion 9 einkoppeln zu können.

Das Meßsystem ist modular aufgebaut. Hierdurch kann Spektralbereich, Fasermaterial, Auswertungsalgorithmus und Einbauumgebung in einem weiten Bereich den Erfordernissen neuer Aufgabenstellungen schnell angepaßt werden.

Meßwerterfassung erfolgt in einer für die Untersuchung notwendigen Geschwindigkeit von bis zu 30 Messungen/Sekunde.

Aufgrund der kleinen Fläche der Sensorspitze 13 können in bevorzugter Weise auch mehrere solcher Sensoren in die Rheometergrundplatte integriert werden. Dadurch kann die Entwässerungskinetik auch ortsaufgelöst bestimmt werden.

### Immobilisierungsmessung

Mit dem nachfolgend bezeichneten Gerät und unter den angegebenen Bedingungen wurde mittels des Verfahrens nach der Erfindung der Einfluß des Verdickers Sterocoll^{R} FD auf das Immobilisierungsverhalten einer Papierstreichfarbe mit CaCO₃ als Pigment (Streichfarbe A) bestimmt.

### Rezeptur Streichfarbe A:

70 Gewichtsteile Hydrocarb^{R} 60
   (CaCO₃-Pigment)
30 Gewichtsteile Hydrocarb^{R} 90
   (CaCO₃-Pigment)
10 Gewichtsteile Styronal^{R} LD 615
   (Styrol/Butadien/Acrylnitril-Copolymerdispersion)
x Gewichtsteile Verdicker Sterocoll^{R} FD
   (Acrylester/Acrylsäure-Copolymer)
   Feststoffgehalt: 62 %, pH: 8,5 - 9.

| | |
|---|---|
| Meßinstrument: | Schubspannungskontrolliertes Rotationsrheometer BOHLIN^{R} CS |
| Meßgeometrie: | Platte-Platte |
| Obere Platte: | Edelstahl, Durchmesser 35 mm |
| Grundplatte: | Nitrocellulose-Filter der Firma Schleicher & Schüll, Porengröße 5 µm, Gesamtdurchmesser 50 mm, nutzbarer Durchmesser 42 mm |
| Halterung: | gelochte Messingplatte |
| Spalthöhe: | 200 µm |
| Schubspannung: | 100 Pa |
| Druck: | 250 mbar (unter dem Filterpapier), 950 mbar (über der Papierstreichfarbe) |
| Meßtemperatur: | 25 °C |

Die zeitliche Entwicklung der scheinbaren Viskosität nach Beginn des Entwässerungsvorgangs ist in Figur 3 für die in Tabelle 1 angegebenen Verdickerkonzentrationen dargestellt. t=0 bezeichnet den Beginn der Entwässerung durch Einschalten des Unterdrucks.

Die Viskositätskurven zeigen einen charakteristischen Knick. Aus dem Schnittpunkt der Tangenten an den vorderen beziehungsweise hinteren Ast der jeweiligen Kurven (siehe Figur 3) kann eine charakteristische Immobilisierungszeit tᵢₘₘ bestimmt werden.

Die Zeit tᵢₘₘ steigt im vorliegenden Beispiel mit zunehmender Verdickerkonzentration drastisch an (Tabelle 1).

**Tabelle 1:**

| | |
|---|---|
| Abhängigkeit der Immobilisierungszeit tᵢₘₘ von der Verdickerkonzentration | |
| x (Verdickerkonzentration in %) | t_{imm[s]} |
| 0,4 | 220 |
| 0,6 | 420 |
| 0,8 | 635 |

Es zeigt sich somit, daß in Übereinstimmung mit dem realen Verhalten im Beschichtungsprozeß mit zunehmender Verdickerkonzentration das Wasserrückhaltevermögen dieser Streichfarbenformulierungen deutlich ansteigt.

### Wassergehaltsbestimmung mit faseroptischer NIR-Spektroskopie

Die im Detektor 11 registrierte Lichtintensität wird in Form eines Spannungssignals ausgegeben. Der Wassergehalt der Probe ist mit der Spannungsdifferenz ΔU verknüpft, die sich aus den unterschiedlichen Intensitäten des bei 1470 nm bzw. 1700 nm reflektierten Lichts ergibt.

### Kalibrierung des Spannungssignals:

Für die Erstellung der Kalibrierkurve sind Proben gleicher Rezeptur mit unterschiedlichem Feststoffgehalt notwendig. Für die hier als Beispiel angeführte Streichfarbe B wurden diese Kalibrierproben durch geeignete Verdünnung einer Ausgangsprobe mit einem Feststoffgehalt von 80,5% hergestellt.

### Rezeptur Streichfarbe B:

100 Gewichtsteile Hydrocarb^{R} OG
   (CaCO₃-Pigment
0,4 Gewichtsteile Polysalz^{R} SR
   (Polyacrylsäure-Salz)
0,1 Gewichtsteile NaOH
10 Gewichtsteile Styronal^{R} LD 615
   (Styrol/Butadien/Acrylnitril-Copolymerdispersion)
0,5 Gewichtsteile Sterocoll^{R} FD
   (Acrylester/Acrylsäure-Copolymer)

Der Feststoffgehalt F der einzelnen Proben wurde als Funktion der jeweils gemessenen Spannungsdifferenz ΔU aufgetragen (Figur 4). Durch Anpassung eines Polynoms 2. Grades an die so bestimmten Wertepaare wurde eine (für die Streichfarbe B gültige) Kalibrierfunktion bestimmt. Anhand dieser Funktion können die während des Entwässerungsvorgangs gemessenen Spannungsdifferenzen ΔU in Feststoffgehalts-Werte umgerechnet werden.

Die Kalibrierfunktion hängt stark von den Gerätespezifikationen ab und muß bei jeder Modifikation des Meßaufbaus (Alterung/Austausch der Halogenlampe, Änderung/Drift des Meßverstärkers u.ä.) neu bestimmt werden.

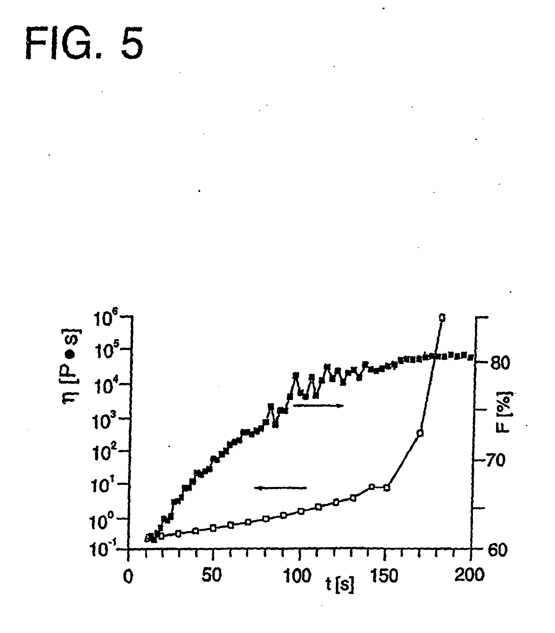

Das Ergebnis der simultanen Bestimmung des Feststoffgehalts F ■ und der scheinbaren Viskosität η □ als Funktion der Entwässerungszeit für Streichfarbe B ist in Figur 5 dargestellt, wobei mitt=der Beginn der Entwässerung durch Einschalten des Unterdrucks bezeichnet ist.

## Patentansprüche

1. Verfahren zur Bestimmung der Immobilisierung kolloider Beschichtungsdispersionen (9) beim Aufbringen auf ein poröses Substrat (5) durch Viskositätsmessung, wobei auf das poröse Substrat (5), das eine Begrenzungsfläche des Meßspalts eines Rheometers bildet, die kolloide Beschichtungsdispersion (9) aufgebracht und die zeitliche Änderung der Viskosität gemessen wird.

2. Verfahren nach Anspruch 1, **dadurch gekennzeichnet, daß** die zeitliche Änderung der Viskosität unter Vorgabe einer konstanten Schubspannung oder Schergeschwindigkeit gemessen wird.

3. Verfahren nach Anspruch 1 oder 2, **dadurch gekennzeichnet, daß** die Höhe des Meßspalts auf einen Wert zwischen 20µm und 2mm, bevorzugt zwischen 50µm und 500µm eingestellt wird.

4. Verfahren nach einem der vorhergehenden Ansprüche, **dadurch gekennzeichnet, daß** an die der die kolloide Beschichtungsdispersion (9) tragenden Fläche gegenüberliegende Fläche des porösen Substrats (5) ein Unterdruck angelegt wird.

5. Verfahren einem der vorhergehenden Ansprüche, **dadurch gekennzeichnet, daß** über einen Sensor (13), der in die kolloide Beschichtungsdispersion (9) ragt, der zeitliche Verlauf des Verlustes an flüssiger Phase in der kolloiden Beschichtungsdispersion (9) gemessen wird.

6. Verfahren nach Anspruch 5, **dadurch gekennzeichnet, daß** der Sensor (13) Teil eines faseroptischen Streulichtmeßsystems (10, 11, 13) ist.

7. Verfahren nach Anspruch 5, **dadurch gekennzeichnet, daß** das faseroptische Streulichtmeßsystem (10, 11, 13) im NIR-Strahlungsbereich arbeitet.

8. Verwendung des Verfahrens nach einem der vorhergehenden Ansprüche zur Bestimmung der Immobilisierung und gegebenenfalls des Verlustes an flüssiger Phase einer kolloiden Beschichtungsdispersion (9) beim Auftrag auf ein poröses Substrat (5), insbesondere von Papierstreichfarbe beim Auftrag auf Rohpapier.

## Claims

1. A method for determining the immobilization of colloidal coating dispersions (9) on application to a porous substrate (5) by viscosity measurement, wherein the colloidal coating dispersion (9) is applied to the porous substrate (5) which forms a bounding surface of the measuring gap of a rheometer, and the change in the viscosity as a function of time is measured.

2. A method as claimed in claim 1, wherein the change in the viscosity as a function of time is measured with specification of a constant shear stress or shear rate.

3. A method as claimed in claim 1 or 2, wherein the height of the measuring gap is set at from 20 µm to 2 mm, preferably from 50 µm to 500 µm.

4. A method as claimed in any of the preceding claims, wherein reduced pressure is applied to that surface of the porous substrate (5) which is opposite the surface carrying the colloidal coating dispersion (9).

5. A method as claimed in any of the preceding claims, wherein the loss of liquid phase in the colloidal coating dispersion (9) as a function of time is measured by means of a sensor (13) which projects into the colloidal coating dispersion (9).

6. A method as claimed in claim 5, wherein the sensor (13) is part of a fiber optic scattered light measuring system (10, 11, 13).

7. A method as claimed in claim 5, wherein the fiber optic scattered light measuring system (10, 11, 13) operates in the NIR radiation range.

8. The use of a method as claimed in any of the preceding claims for determining the immobilization and, if required, the loss of liquid phase of a colloidal coating dispersion (9) on application to a porous substrate (5), in particular of a paper coating slip on application to base paper.

## Revendications

1. Procédé pour déterminer l'immobilisation de dispersions colloïdales de revêtement (9) lors de leur application sur un substrat poreux (5) au moyen d'une mesure de viscosité, dans lequel on applique la dispersion colloïdale de revêtement (9) sur le substrat poreux (5) qui forme la périphérie de la fente de mesure d'un rhéomètre, et dans lequel on mesure la variation de la viscosité dans le temps.

2. Procédé selon la revendication 1, **caractérisé en ce que** l'on mesure la variation de la viscosité dans le temps en imposant une vitesse de cisaillement ou une contrainte de cisaillement constantes.

3. Procédé selon la revendication 1 ou 2, **caractérisé en ce que** l'on choisit une hauteur de la fente de mesure comprise entre 20 µm et 2 mm, de préférence entre 50 µm et 500 µm.

4. Procédé selon une quelconque des revendications précédentes, **caractérisé en ce que** l'on applique une pression inférieure à la pression atmosphérique à la surface du substrat poreux (5) qui est située en face de la surface présentant la dispersion colloïdale de revêtement (9).

5. Procédé selon une quelconque des revendications précédentes, **caractérisé en ce que** l'on mesure l'évolution dans le temps de la perte de phase liquide dans la dispersion colloïdale de revêtement (9), au moyen d'un détecteur (13) qui fait sailli dans la dispersion colloïdale de revêtement (9).

6. Procédé selon la revendication 5, **caractérisé en ce que** le détecteur (13) est un élément d'un système de mesure à fibre optique de la lumière diffusée (10, 11, 13).

7. Procédé selon la revendication 5, **caractérisé en ce que** le système de mesure à fibre optique de la lumière diffusée (10, 11, 13) opère dans le domaine du proche infrarouge (NIR).

8. Mise en oeuvre du procédé selon l'une quelconque des revendications précédentes pour déterminer l'immobilisation et éventuellement la perte de phase liquide dans la dispersion colloïdale de revêtement (9) lors de son application sur le substrat poreux (5), en particulier pour une peinture de couchage pour papier lors de son application sur le papier brut.
